(19) Europäisches Patentamt
European Patent Office
Office européen des brevets

(11) **EP 3 272 713 B1**

(12) **EUROPEAN PATENT SPECIFICATION**

(45) Date of publication and mention
of the grant of the patent:
**09.06.2021 Bulletin 2021/23**

(21) Application number: **15885811.8**

(22) Date of filing: **20.03.2015**

(51) Int Cl.:
*C02F 1/30* (2006.01)        *A61P 3/04* (2006.01)
*A61P 3/10* (2006.01)        *A61P 9/12* (2006.01)
*C02F 1/00* (2006.01)        *A61K 41/00* (2020.01)
*C02F 1/48* (2006.01)

(86) International application number:
**PCT/CN2015/074742**

(87) International publication number:
**WO 2016/149872 (29.09.2016 Gazette 2016/39)**

(54) **PREPARATION METHOD OF DRINKING WATER**

HERSTELLUNGSVERFAHREN FÜR TRINKWASSER

PROCÉDÉ DE PRÉPARATION D'EAU POTABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**24.01.2018 Bulletin 2018/04**

(73) Proprietor: **Happy Ocean (Beijing) Water
Technology Co.,Ltd.
Beijing 101105 (CN)**

(72) Inventor: **WANG, Weixing
Beijing 101105 (CN)**

(74) Representative: **Schiweck Weinzierl Koch
Patentanwälte Partnerschaft mbB
Ganghoferstraße 68 B
80339 München (DE)**

(56) References cited:
**CN-A- 1 748 856          CN-A- 101 046 686
CN-A- 101 466 643        CN-A- 103 058 430
CN-A- 104 720 058        CN-A- 104 757 672
CN-A- 104 757 673        CN-A- 104 757 674
CN-A- 104 757 675        CN-A- 104 784 207
CN-A- 104 803 440        CN-U- 202 924 802
CN-Y- 2 635 659          CN-Y- 2 758 237
CN-Y- 2 778 796          JP-A- H02 131 186
KR-A- 20080 083 789      US-A1- 2004 050 682
US-A1- 2007 221 577      US-A1- 2009 242 407**

• **ZHANG, JIANPING ET AL.: 'Structural Change of
Water Clusters and the Corresponding Biological
Effects.' CHEMISTRY vol. 67, no. 04, 18 April 2004,
XP009501306**

Remarks:
The file contains technical information submitted after
the application was filed and not included in this
specification

**Description**

TECHNICAL FIELD

The present invention relates to the field of water treatment technology and more particularly to a multi-polar micro-kinetic energy drinking water and preparation method for same and use thereof.

BACKGROUND OF THE INVENTION

Water ($H_2O$) is an inorganic substance consisting of two elements of hydrogen and oxygen, and is a colorless, odorless and transparent liquid at normal temperature and pressure. Water is one of the most common substances and it is an important resource for the survival of all life, including human beings and it is also the most important part of the organism. Water plays an important role in the evolution of life. Humans began to generate awareness on the water very early, in the ancient East-West simple material concept, water was regarded as a basic constituent element.

Modern people have the following cognitions on water from a scientific angle:
Water molecules have memory: when scientists melted and then refreezed up individual snow crystals with different shapes and weights, it was found that the shape and weight of the re-frozen snow crystals were exactly the same as that before melting and therefore, scientists come to the conclusion that the water itself has "memory".

Since the water molecule itself has its own special structural shape, it is made up of two hydrogens and one oxygen ion at an angle of 104.5. It is a kind of ionization system with electronic "magnetic polarity". Water molecule at zero and below exhibits the state of solid crystal, and we call it ice. However water is not completely liquid at a temperature of 0 to 60 °C, but in the form of a liquid crystal. But if the temperature is higher than 60 °C, this liquid crystal molecule will be destroyed.

Due to the special angle of the water molecule, the body of the diluted solute molecule can be contained in its liquid crystal, which is the general understanding of the dissolution process. At present, new finding is that water molecules are still able to keep the "shape" memory on the solute molecules when the solute molecules are separated from the water molecules by some means (such as by repeated dilution and shock). When the body cells are exposed to such water molecules, due to the "shape" memory on the solute molecule they will be treated as true solute molecules, this is the cornerstone of homeopathic therapy for more than 200 years.

Water is composed of two elements of hydrogen and oxygen, it does not exsit in the form of individual water molecules, but in the form of water molecules group $(H_2O)_n$ (i.e. the liquid crystal described above), which is formed by gathering many water molecules in nature. The water molecules group may be chain-like, ring-like, slug-like or grape-like, and have at least five, or as many as more than ten, dozens to hundreds of water molecules.

US 2009/0242407 A1 discloses an electromagnetic field treatment method and electromagnetic field treatment equipment of water.

US 2007/221577 A1 discloses a method for electromagnetic treatment of water conferring a biological activity thereon.

US 2004/0050682 A1 discloses an activated water apparatus and methods and products.

There are already some methods for treating water molecules to increase the energy level of water molecules or disinfect or prevent and remove dirts by using electromagnetic waves. However, all of these methods can not adequately control the treatment effect, and the treated water by such methods is mostly used as the drinking water without other additional beneficial effects.

SUMMARY OF THE INVENTION

In order to overcome the drawbacks of the prior art, the present invention provides a method for prepation of a drinking water, comprising three different frequencies of electromagnetic waves: low, medium and high frequency electromagnetic waves, were applied at the outside of the pipe of raw water of ordinary drinking water in non-contact mode, wherein the frequency of the low frequency electromagnetic wave was 45 kHz, the frequency of the medium frequency electromagnetic wave was 600 kHz, and the frequency of the high frequency electromagnetic wave was 300 MHz;
wherein, the low frequency electromagnetic wave and the medium frequency electromagnetic wave were both sine wave and they were propagated in the same direction, the high frequency electromagnetic wave was triangular wave, and its propagation direction was perpendicular to that of the low frequency electromagnetic wave and the medium frequency electromagnetic wave, when treatment, firstly, the low frequency electromagnetic wave and the high frequency electromagnetic wave were used simultaneously for 15 minutes, and then the medium electromagnetic wave and the high frequency electromagnetic wave were used simultaneously for 15 minutes, after the treatment was completed, the multi-polar micro-kinetic energy drinking water was obtained.

Compared with the raw water of drinking water, the intensity of the fluorescence emission spectrum of the multi-polar micro-kinetic energy drinking water is greatly improved in the spectral range between 300nm and 400nm. The results from many repeated experiments on the same experimental sample show that the experiment has good reproducibility.

The experiments show that the multi-polar micro-kinetic energy drinking water of the present invention has more extranuclear electrons in the non-radiative high energy level.

Preferably, the raw water of drinking water is ordinary tap water or various mineral water or purified water such as Evian Natural Mineral Water, Tibet Glacier Mineral Water, NongFu Spring, Wahaha and so on.

It is best to drink or use the multi-polar micro-kinetic energy drinking water of the present invention within 72 hours after preparation.

Compared with raw water of drinking water, the intensity of the fluorescence emission spectrum of the multi-polar micro-kinetic energy drinking water prepared by the method of the present invention is greatly improved in the spectral range between 300nm and 400nm. The results from many repeated experiments on the same experimental sample show that the experiment has good reproducibility.

The experiments show that the multi-polar micro-kinetic energy drinking water of the present invention has more extranuclear electrons in the non-radiative high energy level.

In the present invention, the method and apparatus for generating the electromagnetic waves are conventional technical means in the art.

It is further disclosed herein a use of the multi-polar micro-kinetic energy drinking water described above for the preparation of various mineral water, purified water, beverages, health care products or medicaments.

Preferably, the beverage is a functional beverage and can be used for inhibiting fatigue, losing weight, defaecating, lowering blood pressure, lowering blood glucose, lowering blood uric acid and lowering blood urea.

Preferably, the health care products or medicaments can be used for inhibiting fatigue, losing weight, defaecating, lowering blood pressure, lowering blood glucose, lowering blood uric acid and lowering blood urea.

Compared with the prior art, the present invention treats water using the electromagnetic wave non-contact treatment water without adding any additives, the preparation method of the present invention is simple and has high production efficiency and low cost.

Compared with the existing water, the ultraviolet absorption peak of the multi-polar micro-kinetic energy drinking water prepared by the method of the present invention is shifted obviously to the short-wave direction relative to the untreated raw water of drinking water, and the intensity of the fluorescence emission spectrum of the multi-polar micro-kinetic energy drinking water is greatly improved in the spectral range between 300nm and 400nm, and more extranuclear electrons are in the non-radiative high energy level. Animal experiments have proven that the multi-polar micro-kinetic energy drinking water prepared by the method of the present invention has the following effects: inhibiting fatigue, losing weight, defaecating, lowering blood pressure, lowering blood glucose, lowering blood uric acid and lowering blood urea.

## BRIEF DESCRIPTION OF THE DRAWINGS

Hereinafter, exemplary embodiments of the present invention will be described in detail with reference to the accompanying drawings, wherein:

Figure 1 shows the effect of the water from different treatment groups on DNA damage induced by peroxide;

Figure 2 shows the effect of the culture medium from different treatment groups on cells;

Figure 3 shows the absorption spectrum of raw water of ordinary drinking water and the multi-polar micro-kinetic energy water prepared according to the present invention obtained from the raw water of ordinary drinking water.

Figure 4 shows the absorption spectrum distribution of Evian Natural Mineral Water (raw water) and the multi-polar micro-kinetic energy water prepared according to the method of the present invention obtained from the Evian Natural Mineral Water.

Figure 5 shows the absorption spectrum distribution of Tibet Glacier Mineral Water (raw water) and the multi-polar micro-kinetic energy water according to the present invention obtained from the Tibet Glacier Mineral Water.

Figure 6 shows the fluorescence steady-state and transient result of ordinary drinking water (raw water) and the multi-polar micro-kinetic energy drinking water obtained by the preparation method of the present invention from the ordinary drinking water;

Figure 7 shows the fluorescence steady-state and transient result of Evian Natural Mineral Water (raw water) and the multi-polar micro-kinetic energy drinking water obtained by the preparation method of the present invention from the Evian Natural Mineral Water;

Figure 8 shows the fluorescence steady-state and transient result of Tibet Glacier Mineral Water (raw water) and the multi-polar micro-kinetic energy drinking water obtained by the preparation method of the present invention from the Tibet Glacier Mineral Water;

Figure 9 shows the results of repeatability tests of the multi-polar micro-kinetic energy drinking water prepared by the preparation method of the present invention from Tibet Glacier Mineral Water as the raw water in triplicate;
Figure 10 shows the effect of the multi-polar micro-kinetic energy drinking water prepared by the preparation method of the present invention on blood glucose level in normal ICR mice.

## DETAILED DESCRIPTION OF THE EMBODIMENTS

The invention will now be described in further detail with reference to specific embodiments thereof, and the invention is given by way of illustration only and is not intended to limit the scope of the invention.

The experimental methods in the following examples are conventional methods unless otherwise specified. The raw materials, reagents and the like used in the following examples are commercially available unless otherwise specified.

### Example 1 A preparation method of the multi-polar micro-kinetic energy drinking water (not falling under the claimed invention)

Three different frequencies of electromagnetic waves: low, medium and high frequency electromagnetic waves, were applied at the outside of the pipe of raw water of ordinary drinking water in non-contact mode, wherein the frequency of the low frequency electromagnetic wave was 30 kHz, the frequency of the medium frequency electromagnetic wave was 550 kHz, and the frequency of the high frequency electromagnetic wave was 300 MHz.

Wherein, the low frequency electromagnetic wave and the medium frequency electromagnetic wave were both sine wave and they were propagated in the same direction. The high frequency electromagnetic wave was triangular wave, and its propagation direction was perpendicular to that of the low frequency electromagnetic wave and the medium frequency electromagnetic wave. When treatment, firstly, the low frequency electromagnetic wave and the high frequency electromagnetic wave were used simultaneously for 20 minutes, and then the medium electromagnetic wave and the high frequency electromagnetic wave were used simultaneously for 20 minutes. After the treatment was completed, multi-polar micro-kinetic energy drinking water was obtained.

### Example 2 A preparation method of the multi-polar micro-kinetic energy drinking water (not falling under the claimed invention)

Three different frequencies of electromagnetic waves: low, medium and high frequency electromagnetic waves, were applied at the outside of the pipe of raw water of ordinary drinking water in non-contact mode, wherein the frequency of the low frequency electromagnetic wave was 100 kHz, the frequency of the medium frequency electromagnetic wave was 720 kHz, and the frequency of the high frequency electromagnetic wave was 725 MHz.

Wherein, the low frequency electromagnetic wave and the medium frequency electromagnetic wave were both sine wave and they were propagated in the same direction. The high frequency electromagnetic wave was triangular wave, and its propagation direction was perpendicular to that of the low frequency electromagnetic wave and the medium frequency electromagnetic wave. When treatment, firstly, the low frequency electromagnetic wave and the high frequency electromagnetic wave were used simultaneously for 10 minutes, and then the medium electromagnetic wave and the high frequency electromagnetic wave were used simultaneously for 10 minutes. After the treatment was completed, multi-polar micro-kinetic energy drinking water was obtained.

### Example 3 A preparation method of the multi-polar micro-kinetic energy drinking water of the present invention

Three different frequencies of electromagnetic waves: low, medium and high frequency electromagnetic waves, were applied at the outside of the pipe of raw water of ordinary drinking water in non-contact mode, wherein the frequency of the low frequency electromagnetic wave was 45 kHz, the frequency of the medium frequency electromagnetic wave was 600 kHz, and the frequency of the high frequency electromagnetic wave was 300 MHz.

Wherein, the low frequency electromagnetic wave and the medium frequency electromagnetic wave were both sine wave and they were propagated in the same direction. The high frequency electromagnetic wave was triangular wave, and its propagation direction was perpendicular to that of the low frequency electromagnetic wave and the medium frequency electromagnetic wave. When treatment, firstly, the low frequency electromagnetic wave and the high frequency electromagnetic wave were used simultaneously for 15 minutes, and then the medium electromagnetic wave and the high frequency electromagnetic wave were used simultaneously for 15 minutes. After the treatment was completed, the multi-polar micro-kinetic energy drinking water of the present invention was obtained.

### Example 4 A preparation method of the multi-polar micro-kinetic energy drinking water (not falling under the claimed invention)

Three different frequencies of electromagnetic waves: low, medium and high frequency electromagnetic waves, were applied at the outside of the pipe of raw water of ordinary drinking water in non-contact mode, wherein the frequency of the low frequency electromagnetic wave was 75 kHz, the frequency of the medium frequency electromagnetic wave was 720 kHz, and the frequency of the high frequency electromagnetic wave was 425 MHz.

Wherein, the low frequency electromagnetic wave and the medium frequency electromagnetic wave were both sine wave and they were propagated in the same direction. The high frequency electromagnetic wave was triangular wave, and its propagation direction was perpendicular to that of the low frequency electromagnetic wave and the medium frequency electromagnetic wave. When treatment, firstly, the low frequency electromagnetic wave and the high frequency electromagnetic wave were used simultaneously for 20 minutes, and then the medium electromagnetic wave and the high frequency electromagnetic wave were used simultaneously for 20 minutes. After the treatment was completed, multi-polar micro-kinetic energy drinking water was obtained.

### Example 5 Physical and chemical properties of the multi-polar micro-kinetic energy drinking water

In accordance with the "Standard examination methods for drinking water" (GB/T 5750-2006), the multi-polar micro-kinetic drinking water according to examples 1 to 4 (example 3 being according to the claimed invention) was tested and the testing results were basically the same. The results are shown in Table 1.

Table 1 physical and chemical properties of the multi-polar micro-kinetic energy drinking water

| Nos. | Testing items | Units | National standards | Testing results | Conclusions |
|---|---|---|---|---|---|
| 1 | Chroma | Degree | ≤15 | <5 | Qualified |
| 2 | Turbidity | NTU | ≤1 | 0.39 | Qualified |
| 3 | Smell and taste | Description | none | none | Qualified |
| 4 | Visible material to the naked eye | Description | none | none | Qualified |
| 5 | pH | | 6.5-8.5 | 8.00 | Qualified |
| 6 | Total hardness | mg/L | ≤450 | 164 | Qualified |
| 7 | Aluminum (Al) | mg/L | ≤0.2 | <0.01 | Qualified |
| 8 | Iron (Fe) | mg/L | ≤0.3 | 0.089 | Qualified |
| 9 | Manganese (Mn) | mg/L | ≤0.1 | 0.0082 | Qualified |
| 10 | Copper (Cu) | mg/L | ≤1 | <0.01 | Qualified |
| 11 | Zinc (Zn) | mg/L | ≤1 | 0.108 | Qualified |
| 12 | Sulfates | mg/L | ≤250 | 87.4 | Qualified |
| 13 | Chlorides | mg/L | ≤250 | 47.3 | Qualified |
| 14 | Total dissolved solids | mg/L | ≤1000 | 396 | Qualified |
| 15 | Oxygen consumption | mg/L | ≤3 | 0.68 | Qualified |
| 16 | Volatile phenols | mg/L | ≤0.002 | <0.002 | Qualified |
| 17 | Anionic synthetic detergents | mg/L | ≤0.3 | <0.10 | Qualified |
| 18 | Cyanides | mg/L | ≤0.05 | <0.002 | Qualified |
| 19 | Fluorides | mg/L | ≤1.0 | 0.50 | Qualified |
| 20 | Arsenic (As) | mg/L | ≤0.01 | <0.0005 | Qualified |
| 21 | Selenium (Se) | mg/L | ≤0.01 | 0.0014 | Qualified |
| 22 | Mercury (Hg) | mg/L | ≤0.001 | <0.00005 | Qualified |
| 23 | Cadmium (Cd) | mg/L | ≤0.005 | <0.0001 | Qualified |

(continued)

| Nos. | Testing items | Units | National standards | Testing results | Conclusions |
|---|---|---|---|---|---|
| 24 | Chromium (hexavalent) (Cr(VI)) | mg/L | ≤0.05 | <0.004 | Qualified |
| 25 | Nitrate nitrogen | mg/L | ≤10 | 0.08 | Qualified |
| 26 | Lead (Pb) | mg/L | ≤0.01 | <0.0005 | Qualified |
| 27 | Trichloromethane | mg/L | ≤60 | <0.5 | Qualified |
| 28 | Carbon Tetrachloride | mg/L | ≤2 | <0.1 | Qualified |

According to the above table, it can be seen that the micro-kinetic energy drinking water meet the requirements of "Standard examination methods for drinking water" (GB/T 5750-2006).

**Example 6 Toxicology experiment of the multi-polar micro-kinetic energy drinking water of the present invention**

**I. Acute oral toxicity test**

1. Materials and Methods

1.1 Test substance: the micro-kinetic drinking water prepared in Example 3

1.2 Animals: Kunming mice, which were obtained from Institute of Laboratory Animal Resources, National Institute for Food and Drug Control (certificate number: SCXK (Beijing) 2009-0017), SPF level, weight 18.0-22.0 g; before testing, the animals were fasted overnight and allowed to intake water freely.

1.3 Groups according to dose: the dose of the test substance was 20000 mg/kg body weight, 10 female animals and 10 male animals in each group. They were caged by sex to feed. They were infected by a single oral gavage at a dose of 0.1ml/10g body weight.

1.4 Observations: after infection, the animal's general situation, infected symptoms and death were observed. The observation period was two weeks. At the end of the trial, all animals were dissected to record the general pathological changes. Acute toxicity was graded according to the acute toxicity rating criteria.

2. Test results

Test results of acute oral toxicity test in mice

| Sex | Dose (mg/kg) | Number of Animals | Number of Dead Animals | Mortality Rate (%) |
|---|---|---|---|---|
| Female | 20000 | 10 | 0 | 0 |
| Male | 20000 | 10 | 0 | 0 |

3. Conclusion

After infecntion, the animals do not show any infected symptoms and have no death and no significant pathological changes, and on the contrary, the weight of the animals showed an increasing trend. Therefore, the $LD_{50}$ of the test substance on female and male mice by oral was greater than 10000 mg/kg body weight, so the test substance belongs to the actual non-toxic level.

**II. Effect on DNA damage induced by peroxide**

1. Experimental materials and equipment

1.1 5mM Vitamin C solution: it was prepared by dissolving it in three distilled water and it needs to be prepared before use. Vitamin C powder was obtained from Sigma-Aldrich;

1.2 5mM $CuCl_2$ solution: it was prepared by dissolving it in three distilled water and it needs to be prepared before use. $CuCl_2$ powder was obtained from Sinopharm Chemical Reagent Co. Ltd.;

1.3 Plasmid DNA: it was obtained by amplifing and extracting the pET28a plasmid from DH5a Escherichia coli (the plasmid extraction kit was purchased from Promega);

1.4 5mMEDTA solution, pH8.0: EDTA was obtained from Sinopharm Chemical Reagent Co. Ltd.;

1.5 10×PBS buffer: 80g NaCl, 2g KCl, 14.4g $Na_2HPO_4$ and 2.4g $KH_2PO_4$ were dissolved in distilled water, the pH was adjusted to 7.4 and the volume was adjusted to 1L. The required reagents were purchased from Sinopharm Chemical Reagent Co. Ltd.;

1.6 Agarose: obtained from Biowest;

1.7 Goldview nucleic acid dyes were obtained from Biotium;

1.8 Gel Imager: obtained from Sage Creation Science And Technology Co., Ltd;

1.9 Electrophoresis apparatus: LiuYi instrument factory;

1.10 Test substance: the micro-kinetic drinking water of Examples 3 and 4

2. Experimental methods

2.1 Group of Experiments

Group I: untreated group, the untreated sterile water was applied to the reaction system;
Group II: the micro-kinetic drinking water of example 3;
Group III: the micro-kinetic drinking water of example 4.

The reaction substances were added according to the following order, the final volume of the reaction system was 10 $\mu$L:

10×PBS buffer 1 $\mu$L;
Plasmid DNA (2 $\mu$g) 2 $\mu$L;
5 mM Vitamin C 1 $\mu$L;
5 mM $CuCl_2$ 1 $\mu$L;

supplemented with the untreated water or treated water to 10 $\mu$L.

2.2 The reaction was terminated by adding 1$\mu$L of 5 mM EDTA and 2.2 $\mu$L of loading buffer at 0.5 hour and 1 hour respectively;

2.3 0.8% agarose gel (containing Goldview dye), electrophoresis 1 h;

2.4 Gel imaging.

3. Experimental results

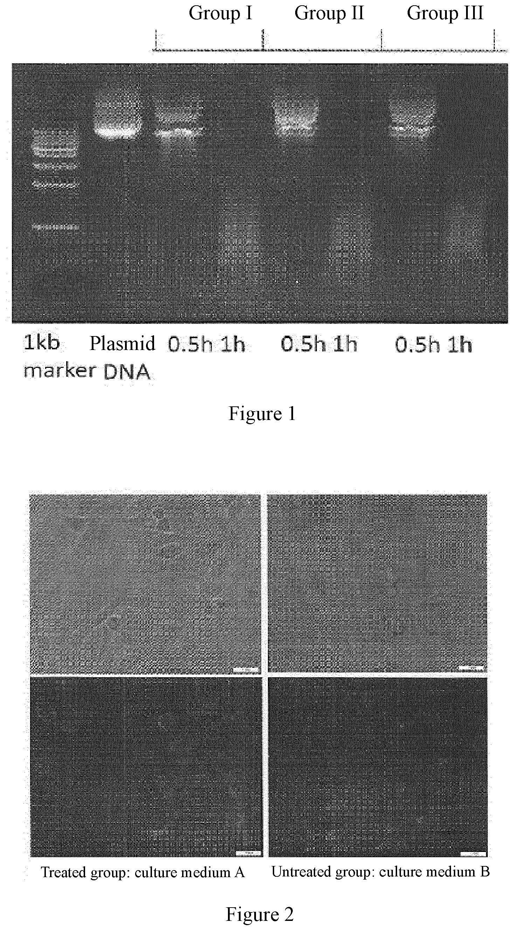

Vitamin C and divalent copper ions occur redox reaction to form peroxide, which will cause plasmid DNA damage and fracture, this embodies in the weakening of the electrophoretic bands. The experimental results are shown in figure 1, the experiment is divided into three groups, the grouping method was as described above, each group is reacted at two time points: 0.5 hours and 1 hour, the brightness of DNA represents DNA content. According to the experimental results, the degradation rate of plasmid DNA by redox reaction was basically the same in group II and III compared with untreated group I, and the micro-kinetic drinking water does not prevent or accelerate DNA damage and fracture.

4. Analysis on the results

Vitamin C and divalent copper ions occur redox reaction to form peroxide, which will cause plasmid DNA damage and fracture, this embodies in the weakening of the electrophoretic bands. The experimental results showed the degradation rate of plasmid DNA by redox reaction was basically the same in group II and III compared with untreated group I, and the micro-kinetic drinking water did not prevent or accelerate DNA damage and fracture. It is shown that the multi-polar micro-kinetic energy water of the present invention has no effect on DNA damage induced by peroxide.

**III. Effect on cell membrane integrity of HepG2 cultured in vitro**

1. Experimental materials and equipment

    1.1 Human hepatocellular carcinoma cells: HepG2;
    1.2 Cell culture medium: DMEM culture medium+10%FBS;
    1.3 FAM-aptamer: purchased from Sangon Biotech (Shanghai) Co., Ltd.;
    1.4 Fluorescent EZ micromirrors: OLYMPUS;
    1.5 96-well cell culture plate;

2. Experimental methods

    2.1 A proper amount of pancreatic enzyme was added to digest the cells in logarithmic growth phase. The cell concentration was adjusted, and the cell was seeded in 96-well plates at 4000 cells per well and cultured overnight;
    2.2 5 ml of DMEM culture medium containing 10% FBS (culture medium A) was treated in the same manner as in Example 3 for 40 minutes (treatment group);
    2.3 5 ml of DMEM culture medium containing 10% FBS (culture medium B) was treated for 40 minutes at the position which was more than 2m away from the wave source of example 3 (untreated group);
    2.4 4 $\mu$g of FAM-labeled C6-8 aptamer was added to 100 $\mu$L of culture medium A and culture medium B respectively and mixed uniformly;
    2.5 The cell fluid adherent cultured in the 96-well plate were replaced with culture medium A and culture medium B containing FAM-C6-8 aptamer, incubated at 37 °C for 30 min, and then washed once with PBS and observed under the fluorescence microscope.

3. Experimental results

The results were shown in figure 2, there was no difference in cell morphology between the treated and untreated groups, the cells in the two groups showed autofluorescence and had intact cell membranes, and no apparent FAM-labeled C6-8 aptamer green fluorescence was observed in the cells.

4. Results and analysis

If the cells are perforated, the FAM-labeled C6-8 aptamer can enter the cell and bind with the protein in the cell and emit green fluorescence upon excitation at 530nm wavelengths. The results show that there is no difference between the cells in the treatment group and that in the untreated group, the cells in the two groups showed autofluorescence, which indicates that the cell membrane is intact. The multi-polar micro-kinetic energy drinking water of the present invention has no effect on cell morphology and cell membrane integrity.

**Example 7 Wide spectral absorptance and UV absorptance of the multi-polar micro-kinetic energy drinking water of the present invention**

1. Experimental materials and equipment

    1.1 The micro-kinetic energy drinking water prepared in Example 3
    1.2 The raw water of ordinary drinking water used in Example 3;
    1.3 The drinking water prepared from Evian Natural Mineral Water, which was treated in the same manner as in Example 3;
    1.4 Evian Natural Mineral Water: purchased from Carrefour supermarket;
    1.5 The drinking water prepared from Tibet Glacier Mineral Water, which was treated in the same manner as in Example 3;
    1.6 Tibet Glacier Mineral Water: purchased from Carrefour supermarket;
    1.7 Ultra-pure water: prepared by the German Sartorius ultra-pure water preparation system;
    1.8 Fiber optic spectrometer Ava-Spec3648, the measuring range is 200-1100 nm, the uncertainty/accuracy is 0.5nm;

1.9 Oscilloscope MS04104, equipment factory number: C001163, measurement accuracy: 1GHz;

1.10 Photodetector Thorlab DET10A/M, equipment factory number: JGZX-ZXZC-012, measurement accuracy: 200 nm-1100 nm;

1.11 Test conditions: the temperature is 21 °C, the humidity is 38%.

2. Experimental methods

2.1 Wide spectrum absorptance

(1) Rinsing the experimental vessels by using about 200 ml ultra-pure water till the physical and chemical indexes were stable.

(2) Placing the raw water of ordinary drinking water used in Example 3 and the micro-kinetic energy drinking water prepared in Example 3 in an experimental vessel, and scanning the spectral absorption of the standard light source of sample at 200 nm to 800 nm, and the experimental results were recorded.

(3) Rinsing the experimental vessels by using about 200 ml ultra-pure water till the physical and chemical indexes restored the original value and reached stability.

(4) Placing the Evian Natural Mineral Water and the drinking water prepared from the Evian Natural Mineral Water by using the same manner as Example 3 in the experimental vessels, repeating the step (2), and then recording the changes in UV intensity during the experimental period.

(5) Rinsing the experimental vessels by using about 200 ml ultra-pure water till the physical and chemical indexes restored the original value and reached stability.

(6) Placing the Tibet Glacier Natural Mineral Water and the drinking water prepared from the Tibet Glacier Natural Mineral Water by using the same manner as Example 3 in the experimental vessels, repeating the step (2), and then recording the changes in UV intensity during the experimental period.

(7) Rinsing the experimental vessels by using about 200 ml ultra-pure water till the physical and chemical indexes restored the original value and reached stability.

2.2 UV absorptance

(1) Rinsing the experimental vessels by using about 200 ml ultra-pure water till the physical and chemical indexes were stable.

(2) Placing the micro-kinetic energy drinking water prepared in example 3 in an ultraviolet intensity detection system, irradiatting the water to be measured using the ultraviolet light source, observaing the ultraviolet intensity value of the water to be measured through the oscilloscope MS04104 and the photodetector Thorlab DET10A/M for 72 hours continuously, and recording the changes in UV intensity during the experimental period.

(3) Rinsing the experimental vessels by using about 200 ml ultra-pure water till the physical and chemical indexes restored the original value and reached stability.

(4) Placing the Evian Natural Mineral Water and the drinking water prepared from the Evian Natural Mineral Water by using the same manner as Example 3 in the ultraviolet intensity detection system, repeating the step (2), and then recording the changes in UV intensity during the experimental period.

(5) Rinsing the experimental vessels by using about 200 ml ultra-pure water till the physical and chemical indexes restored the original value and reached stability.

(6) Placing the Tibet Glacier Natural Mineral Water and the drinking water prepared from the Tibet Glacier Natural Mineral Water by using the same manner as Example 3 in the the ultraviolet intensity detection system, repeating the step (2), and then recording the changes in UV intensity during the experimental period.

(7) Rinsing the experimental vessels by using about 200 ml ultra-pure water till the physical and chemical indexes restored the original value and reached stability.

3. Experimental results

3.1 Wide spectrum absorptance test

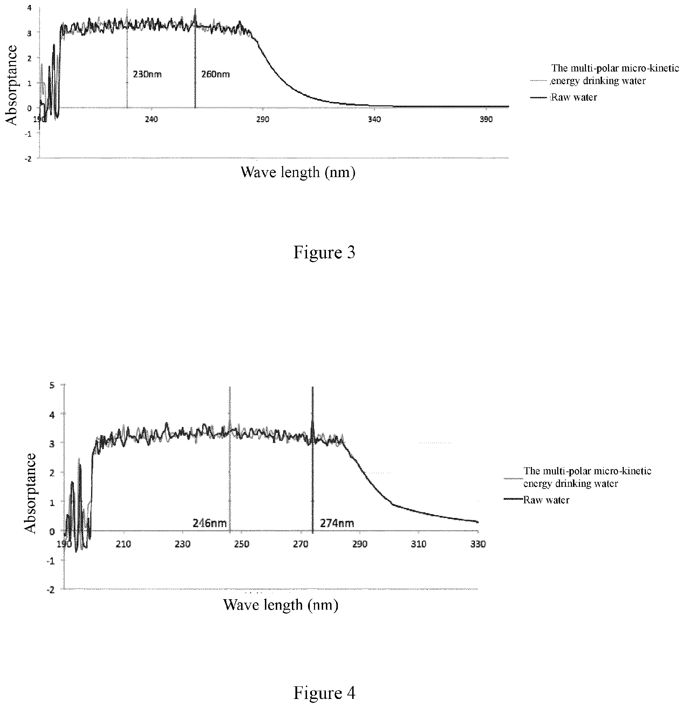

The results are shown in Figure 3-5.

3.2 UV absorptance test

The multi-polar micro-kinetic energy drinking water prepared from ordinary water:
UV absorptance 0.9135 -> 0.9037

The multi-polar micro-kinetic energy drinking water prepared from Evian Natural Mineral Water:
UV absorptance 0.9244 -> 0.9188

The multi-polar micro-kinetic energy drinking water prepared from Tibet Glacier Mineral Water:
UV absorptance 0.9157 -> 0.9079

4. Experimental conclusions and analysis

Through the above experiments, the following conclusions are obtained:

(1) Compared with the raw water, the absorption peak of the absorption spectrum of the multi-polar micro-kinetic energy drinking water of the present invention is shifted to the short wave by about 30 nm;

(2) The absorbance of the multi-polar micro-energy drinking water of the present invention decreased by 1% after testing for 72 hours.

**Example 8 The stimulated fluorescence spectrum change of the multi-polar micro-kinetic energy drinking water of the present invention**

1. Experimental materials and equipment

1.1 The micro-kinetic energy drinking water prepared in Example 3

1.2 The raw water of ordinary drinking water used in Example 3;

1.3 The drinking water prepared from Evian Natural Mineral Water, which was treated in the same manner as in Example 3;

1.4 Evian Natural Mineral Water: purchased from Carrefour supermarket;

1.5 The drinking water prepared from Tibet Glacier Mineral Water, which was treated in the same manner as in Example 3;

1.6 Tibet Glacier Mineral Water: purchased from Carrefour supermarket;

1.7 Ultra-pure water: prepared by the German Sartorius ultra-pure water preparation system;

1.8 Fiber optic spectrometer Ava-Spec3648, equipment factory number is 1101230U1, the measuring range is 200-1100 nm, and the uncertainty/accuracy is 0.5nm;

1.9 Test conditions: the temperature is 22 °C, and the humidity is 37 %.

2. Experimental methods

(1) Rinsing the experimental vessels by using about 200 ml ultra-pure water till the physical and chemical indexes were stable.

(2) Placing the micro-kinetic energy drinking water prepared in Example 3 in a fiber-optic spectrometer sample pool, enforcing an excitation pulse on the sample with a 266 nm pulsed ultraviolet laser, and measuring the emission spectrum of the samples using the scanning function on the fiber-optic spectrometer and recording the fluorescence emission spectra of the samples.

(3) Rinsing the experimental vessels by using about 200 ml ultra-pure water till the physical and chemical indexes restored the original value and reached stability.

(4) Placing the raw water of ordinary drinking water used in Example 3 in the fiber-optic spectrometer sample pool, repeating the step (2), and then recording the changes in UV intensity during the experimental period.

(5) Rinsing the experimental vessels by using about 200 ml ultra-pure water till the physical and chemical indexes restored the original value and reached stability.

(6) Placing the drinking water prepared from Evian Natural Mineral Water by using the same manner as in Example 3 in the fiber-optic spectrometer sample pool, repeating the steps (2) and (3), and then recording the experimental results.

(7) Placing the Evian Natural Mineral Water in the fiber-optic spectrometer sample pool, repeating the steps (2) and (3), and then recording the experimental results.

(8) Placing the drinking water prepared from Tibet Glacier Natural Mineral Water by using the same manner as in Example 3 in the fiber-optic spectrometer sample pool, repeating the steps (2) and (3), and then recording the experimental results.

(9) Placing the Tibet Glacier Natural Mineral Water in the fiber-optic spectrometer sample pool, repeating the steps (2) and (3), and then recording the experimental results.

(10) Placing the drinking water prepared from Tibet Glacier Natural Mineral Water in triplicate by using the same manner as in Example 3 in the fiber-optic spectrometer sample pool, repeating the steps (2) and (3), and then recording the experimental results.

3. Experimental results

3.1 Fluorescence steady-state transient test

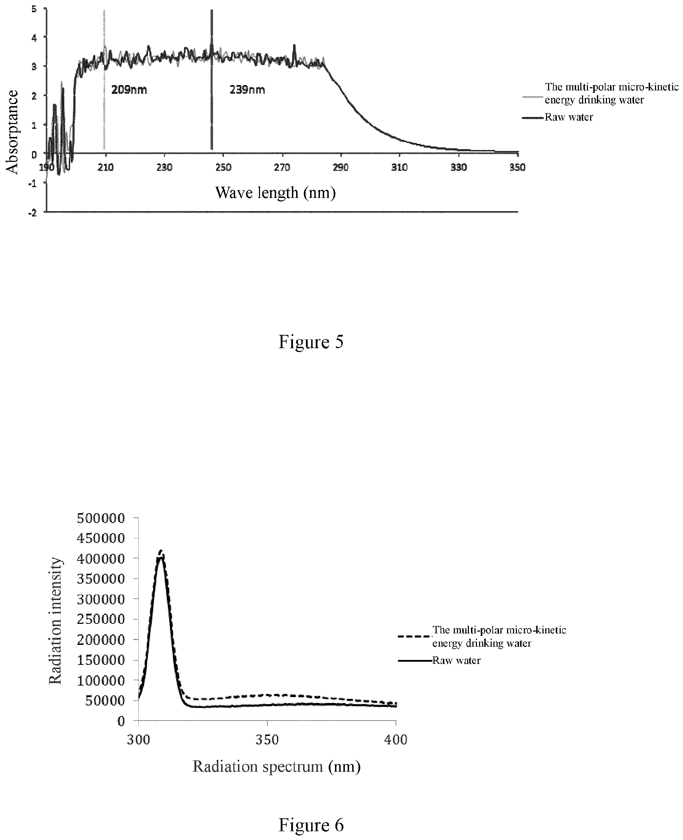

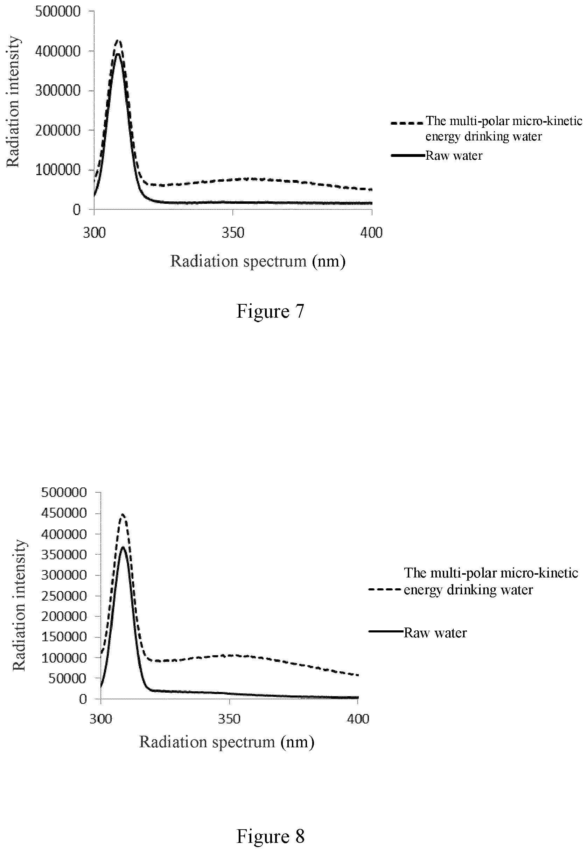

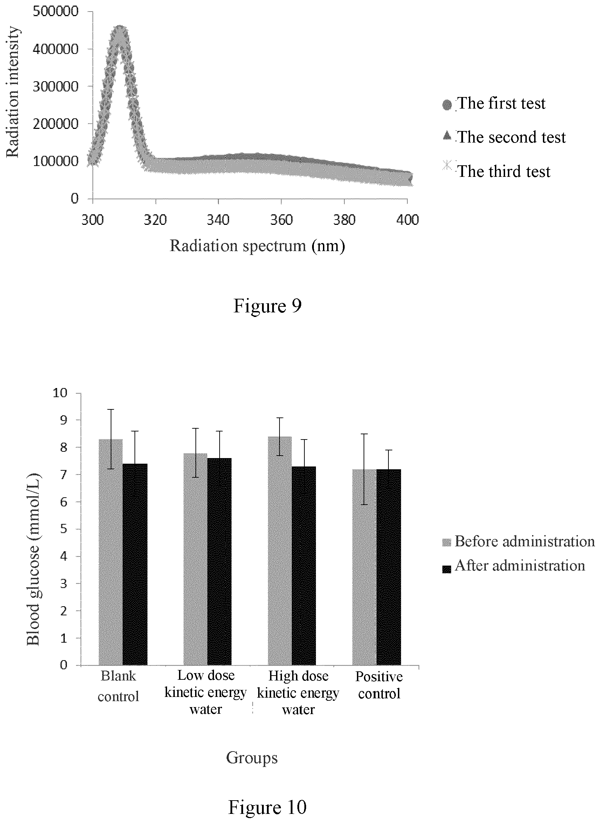

The fluorescence steady-state transient test results of ordinary drinking water and the multi-polar micro-kinetic energy drinking water prepared from the same are shown in Figure 6. The fluorescence steady-state transient test results of Evian Natural Mineral Water and the multi-polar micro-kinetic energy drinking water prepared from the same are shown in Figure 7. The fluorescence steady-state transient test results of Tibet Glacier Mineral Water and the multi-polar micro-kinetic energy drinking water prepared from the same are shown in Figure 8. The repeatability test results of the drinking water prepared from Tibet Glacier Natural Mineral Water in triplicate are shown in Figure 9.

4. Experimental conclusions and analysis

From the above experimental results, it can be seen that compared with raw water of drinking water, the intensity of the fluorescence emission spectrum of the multi-polar micro-kinetic energy drinking water is greatly improved in the spectral range between 300nm and 400nm. The results from many repeated experiments on the same experimental sample show that the experiment has good reproducibility.

The experiments show that the multi-polar micro-kinetic energy drinking water of the present invention has more extranuclear electrons in the non-radiative high energy level.

**Example 9 Effect of the multi-polar micro-kinetic energy drinking water of the present invention on islet-damaged hyperglycemia mice**

1. Materials and Methods

1.1 Samples:

The multi-polar micro-kinetic energy drinking water (the kinetic energy water) prepared in Example 3, which is a colorless, tasteless and transparent water-like liquid, stored at room temperature and should be used within 72 hours after being prepared.

Metformin hydrochloride enteric-coated tablets, which were obtained from Beijing Zhonghui Pharmaceutical Co. Ltd., the batch number is 20140301.

1.2 Experimental animals: ICR mice purchased from Beijing Huafu Kang Biotechnology Co. Ltd., the production license number is SCXK (Beijing) 2014-0004, the laboratory animal quality certificate number is 11401300013568. There are 70 in all, all of them are males, the ordering weight is 18-20g.

Feeding conditions: the laboratory animal facilities continue to maintain the barrier environmental standards. The main environmental indicators are as follows: the room temperature is 20 ~ 26 °C, the daily temperature difference is $\leq 4$ °C. The relative humidity is 40 to 70%. The minimum ventilation time is 15 times per hour. Lighting: Dark = 12 h: 12 h. Animals were housed in PC mice rearing cages and the specifications of cages were $294 \times 190 \times 125$ mm$^3$, 5 in each cage, and their space was in accordance with the provisions of GB14925-2010 of the People's Republic of China on the minimum required space for experimental animals. All animals are fed and managed by trained and qualified personnel. The padding and cage are replaced once a week. And the special feed for mice is added every day for animal consumption and the animal diet activities are kept free during the whole feeding process.

Animal welfare: the animal and related treatment used in this test should meet the requirements of animal welfare. Experiments were carried out after being reviewed and approved by the Laboratory's Institutional Animal Care and Use Committee (IACUC), Beijing Jianhao Pharmaceutical Technology Development Co. Ltd.. The specific considerations are as follows:

(1) During the course of the experiment, the animal's panic and pain should be minimized.

(2) Effective anesthesia must be performed in the operation and dissection of experimental animals. Analgesic, targeted care and diet conditioning should be performed based on the actual situation during postoperative recovery period.

(3) Animal disturbances, panic, pain and injure should be avoided in the experiments of fixation of animal. Fixation machine should have a reasonable structure, appropriate specification, should be durable, environmentally friendly and easy to operate. Without prejudice to the experiment, the mandatory restrictions on the animal's body should be reduced to a minimum.

(4) Sample collection: the operation of the sample collection of experimental animals should be carried out in a safe and humane manner (to minimize stress and discomfort caused to animals).

(5) Dying animals, diseased animals and animals with severe toxicity should be promptly reported to the veterinarian and the person in charge to timely treat or euthanize. Without affecting the determination of the experiment results, "humane endpoint" should be chosen to avoid prolonging the time when the animals suffer from pain. It should be euthanized in accordance with the humanitarian principles when the animals are sacrificed. At the scene of the execution, there should be no other animals present. The body can be properly disposed of after confirmation of the death of the animal.

(6) Occupational safety: personal protective measures such as gloves, gowns, masks, goggles and earplugs etc. should be taken during the experiment.

1.3 Dose selection and the administering manner of the test substances:

After adult animals were purchased and adapted to a few days, 15 animals were randomly taken out to fast for 3-5 hours, and the fasting blood glucose was measured as the basal blood glucose level of the batch of animals. Subsequently, 65 animals were fasted for 24 hours (free drinking water) and injected with alloxan (prepared with fresh normal saline in ice-bath, it needs to be prepared before use) to establish a model, the dose by intravenous infusion was 65 mg/kg of body weight of mice. 3 days later, the blood glucose was measured after the animals were fasted for 3-5 hours, if the blood glucose value was greater than 10 mmol/L, then the animal models with hyperglycemia was successfully established.

1.3.1 Hypoglycemic experiment of the animal models with hyperglycemia

10 animals with normal blood glucose were selected as the blank control group, and 50 hyperglycemia animals were divided into 1 model control group and 4 dose groups according to the blood glucose level (10 each group). In the low dose kinetic energy water group, the animals drank the kinetic energy water daily (the kinetic energy water was kept in a drinking bottle, and supplemented once a day); in addition to drinking the kinetic energy water daily, in the high dose kinetic energy water group, the animals were given the kinetic energy water by gavage once a day, the dose was 0.3 ml/10 g; in the positive-drug metformin hydrochloride enteric-coated tablet group, the animals were given metformin hydrochloride enteric-coated tablet by gavage once a day, the administration dose was 300 mg/kg and the administration volume was 0.3 mL/10 g (the daily dosage of metformin hydrochloride enteric-coated tablet per person is 1.8 g, if the weight of a person is 60kg, the dosage on the person is equivalent to 30 mg/kg, if the dosage used in mice is 10 times of that used in the person, the dosage on the mice is equivalent to 300 mg/kg, the administration volume is 0.3 mL/lOg and the concentration of drugs is 10 mg/ml, the drug is prepared with 0.5% CMC-Na); in the group containing both the kinetic energy water and the metformin hydrochloride enteric-coated tablet (metformin hydrochloride + kinetic energy water), the metformin hydrochloride enteric-coated tablet was formulated with the kinetic energy water once every two days, and administered at dose of 300 mg/kg.

$$\text{Blood glucose reduction rate}\% = \frac{(\text{Blood glucose before experiment} - \text{blood glucose after experiment})}{\text{Blood glucose before experiment}} \times 100\%$$

Table 2 Effect of the kinetic energy water of the present invention on the blood glucose level of the islet-damaged hyperglycemia mice

| Group | Dosage | Administration volume | Numbers of animals | Cage number | Serial number |
|---|---|---|---|---|---|
| Blank control group | - | - | 10 | 1,2 | 1-10 |
| Model control group | - | - | 10 | 3,4 | 11-20 |
| Low dose kinetic energy water group | Daily drinking | Daily drinking | 10 | 5, 6 | 21-30 |
| High dose kinetic energy water group | Daily drinking + 0.3 mL/10g | Daily drinking + 0.3 mL/10g | 10 | 7, 8 | 31-40 |
| Metformin hydrochloride group | 300 mg/kg | 0.3 mL/10g | 10 | 9, 10 | 41-50 |
| Kinetic energy water + Metformin hydrochloride | Kinetic energy water + 300 mg/kg | Kinetic energy water+0.3 mL/10g | 10 | 11, 12 | 51-60 |

1.4 Main instruments and reagents:

Roche ACCU-CHEK fast blood glucose meter, special test papers for the blood glucose meter.

1.5 Test methods:

15 mice were selected for blood glucose measurement before modeling, and the blood glucose values were taken as the base values. In addition, blood was collected separately before grouping, on the 15th day of administration, 30th day of administration and 40th day of administration for blood glucose measurement, the blood samples were taken by pricking the tail vein with a needle, and the volume of the collected blood was at least 5 μL.

1.6 Statistics and analysis of the test data

A SPSS statistical software was used to process the results of body weight and compare and analyze the administration and control group. Statistical analysis was done according to the following methods: the normal test was performed using Kolmogorov-Smirnov method and the test for homogeneity of variance was performed using Levene median method, if $P > 0.05$, then one-way ANOVA method will be performed, and if the normal test and the test for homogeneity of variance failed ($P < 0.05$), then a nonparametric Mann-Whitney test will be required.

1.7 Determination of results

*$p < 0.05$ was considered to indicate a statistically difference, **$P < 0.01$ was considered to indicate a statistically significant difference. In addition, the absolute value of the measurement results should be as a reference to determine whether the kinetic energy water and positive-drugs have an impact on the blood glucose levels of normal animals.

2. Results

15 mice were randomly selected for blood glucose measurement and the result was $8.5 \pm 0.8$ mmol/L.

Table 3 Blood glucose results (mmol/L) in ICR mice when modeling and grouping

| Serial number | Blank control | Serial number | Model group | Serial number | Low dose kinetic energy water group |
|---|---|---|---|---|---|
| 1 | 8.9 | 11 | 32.3 | 21 | 31.1 |
| 2 | 7.2 | 12 | 16.4 | 22 | 29.6 |
| 3 | 8 | 13 | 29.8 | 23 | 29.8 |
| 4 | 7.1 | 14 | 28.5 | 24 | 31.3 |
| 5 | 7 | 15 | 30.9 | 25 | 11.3 |

(continued)

| Serial number | Blank control | Serial number | Model group | Serial number | Low dose kinetic energy water group |
|---|---|---|---|---|---|
| 6 | 6.9 | 16 | 31.2 | 26 | 30.9 |
| 7 | 6.9 | 17 | 33 | 27 | 23.4 |
| 8 | 6 | 18 | 30.2 | 28 | 27.5 |
| 9 | 6.6 | 19 | 31.2 | 29 | 30.8 |
| 10 | 6.8 | 20 | 28.5 | 30 | 28.1 |
| Serial number | High dose kinetic energy water group | Serial number | Metformi n hydrochl oride | Serial number | Metformin hydrochloride + kinetic energy water |
| 31 | 23.4 | 41 | 28.4 | 51 | 30.2 |
| 32 | 32.1 | 42 | 29.9 | 52 | 32.4 |
| 33 | 32.3 | 43 | 32.1 | 53 | 32.1 |
| 34 | 14 | 44 | 32.9 | 54 | 32.9 |
| 35 | 30.2 | 45 | 33.2 | 55 | 31.5 |
| 36 | 31.1 | 46 | 32.4 | 56 | 33.1 |
| 37 | 32.8 | 47 | 10.8 | 57 | 18.4 |
| 38 | 33.2 | 48 | 30.4 | 58 | 21.2 |
| 39 | 31.3 | 49 | 27.8 | 59 | 22.7 |
| 40 | 29.5 | 50 | 26.6 | 60 | 33.1 |

Table 4 Blood glucose results in each group of ICR mice when modeling and grouping (mmol/L, $\bar{x} \pm s$, n=10)

| Group | Blank control | Model group | Low dose kinetic energy water group | High dose kinetic energy water group | Metformin hydrochlori de | Metformin hydrochlori de + kinetic energy water |
|---|---|---|---|---|---|---|
| Blood Glucose | 7.1±0.8 | 29.2±4.7 | 27.4±6.1 | 29.0±6.0 | 28.5±6.6 | 28.8±5.7 |

It can be seen from Table 3 and Table 4 that the blood glucose of the blank control group was 7.1 ± 0.8 mmol/L and the blood glucose of the other groups was 29.2 ± 4.7, 27.4 ± 6.1, 29.0 ± 6.0, 28.5 ± 6.6 and 28.8 ± 5.7 mmol/L respectively, this shows that the model was successfully established.

Table 5 Blood glucose results of each group after 15 days of continuous administration (mmol/L)

| Serial number | Blank control | Serial number | Model group | Serial number | Low dose kinetic energy water group |
|---|---|---|---|---|---|
| 1 | 7.9 | 11 | 32.9 | 21 | 31.9 |
| 2 | 7.9 | 12 | >33.3 | 22 | >33.3 |
| 3 | 8.7 | 13 | >33.3 | 23 | 30.9 |
| 4 | 8.3 | 14 | 31.2 | 24 | 31.4 |
| 5 | 7.7 | 15 | 29.8 | 25 | 27.4 |
| 6 | 7.6 | 16 | 31 | 26 | 29.8 |

(continued)

| Serial number | Blank control | Serial number | Model group | Serial number | Low dose kinetic energy water group |
|---|---|---|---|---|---|
| 7 | 8.5 | 17 | >33.3 | 27 | 13.7 |
| 8 | 8.2 | 18 | >33.3 | 28 | 33.1 |
| 9 | 8.5 | 19 | >33.3 | 29 | >33.3 |
| 10 | 8.4 | 20 | 10.4 | 30 | 32.1 |
| Serial number | High dose kinetic energy water group | Serial number | Metformin hydrochloride | Serial number | Metformin hydrochloride + kinetic energy water |
| 31 | 29.8 | 41 | 22.9 | 51 | 27.2 |
| 32 | 32.7 | 42 | 29.5 | 52 | 28.2 |
| 33 | >33.3 | 43 | 23.9 | 53 | 27.5 |
| 34 | 10.1 | 44 | 16.6 | 54 | 27.1 |
| 35 | >33.3 | 45 | >33.3 | 55 | 28.5 |
| 36 | 32.4 | 46 | 21.4 | 56 | 26.3 |
| 37 | 26.7 | 47 | 9.3 | 57 | 16 |
| 38 | >33.3 | 48 | 30.5 | 58 | 6.9 |
| 39 | 30.1 | 49 | 9.4 | 59 | 26.5 |
| 40 | 31.7 | 50 | 25.4 | 60 | 28.7 |

Table 6 Effect of the kinetic energy water of the present invention on the blood glucose level of the islet-damaged hyperglycemia mice after 15 days of administration (mmol/L , $\bar{x} \pm s$, n=10)

| Group | Blank control | Model group | Low dose kinetic energy water group | High dose kinetic energy water group | Metformin hydrochloride | Metformin hydrochloride + kinetic energy water |
|---|---|---|---|---|---|---|
| Blood Glucose | 8.2±0.4 | 30.2±7.1 | 29.7±5.9 | 29.3±7.1 | 22.2±8.3* | 24.3±7.1 |

Remark: * Compared with the model group, p<0.05

It can be seen from Table 5 and Table 6 that the blood glucose level of the blank control group was 8.2 $\pm$ 0.4 mmol/L after 15 days of continuous administration. The blood glucose levels of 5 of the 10 mice (5/10) in the model group, 2 mice (2/10) in the low dose kinetic energy water group, 3 mice (3/10) in the high-dose kinetic energy water group and 1 mouse (1/10) in the metformin hydrochloride group were higher than the upper limit of the glucose meter (33.3 mmol/L) and the metformin hydrochloride + kinetic energy water group were all below the upper limit of the glucose meter. The blood glucose of animals exceeding the upper limit of measurement was calculated in 33.3 mmol/L, the blood glucose results of each group were 30.2 $\pm$ 7.1, 29.7 $\pm$ 5.9, 29.3 $\pm$ 7.1, 22.2 $\pm$ 8.3 and 24.3 $\pm$ 7.1 mmol/L respectively. Compared with the model group, the absolute value of the blood glucose of the metformin hydrochloride group and the metformin hydrochloride and kinetic energy water group decreased, and the metformin hydrochloride group had statistical difference.

Table 7 Blood glucose results of each group after 30 days of continuous administration (mmol/L)

| Serial number | Blank control | Serial number | Model group | Serial number | Lowdose kinetic energy water group |
|---|---|---|---|---|---|
| 1 | 7.1 | 11 | >33.3 | 21 | 25.9 |

(continued)

| Serial number | Blank control | Serial number | Model group | Serial number | Lowdose kinetic energy water group |
|---|---|---|---|---|---|
| 2 | 6.2 | 12 | 32.5 | 22 | 30.5 |
| 3 | 8.2 | 13 | >33.3 | 23 | 25.7 |
| 4 | 7.4 | 14 | 31.1 | 24 | 27.7 |
| 5 | 7.5 | 15 | 31.4 | 25 | 27.9 |
| 6 | 6.3 | 16 | 26.7 | 26 | 16.9 |
| 7 | 8.3 | 17 | >33.3 | 27 | 22.1 |
| 8 | 8.6 | 18 | 17.9 | 28 | 28.6 |
| 9 | 7.5 | 19 | >33.3 | 29 | 24.1 |
| 10 | 8.1 | 20 | 7.4 | 30 | 22.4 |
| Serial number | High dose kinetic energy water group | Serial number | Metformin hydrochloride | Serial number | Metformin hydrochloride + kinetic energy water |
| 31 | 27.9 | 41 | 23.8 | 51 | >33.3 |
| 32 | 24.4 | 42 | 12.5 | 52 | 23.4 |
| 33 | 32.2 | 43 | 15.8 | 53 | 31.7 |
| 34 | 5.4 | 44 | 10.3 | 54 | >33.3 |
| 35 | 32.6 | 45 | 26.7 | 55 | >33.3 |
| 36 | >33.3 | 46 | 25 | 56 | 30.8 |
| 37 | 31.6 | 47 | 7.2 | 57 | 26.2 |
| 38 | 24.4 | 48 | 32.7 | 58 | 5.5 |
| 39 | 25.2 | 49 | 9.8 | 59 | >33.3 |
| 40 | 25.9 | 50 | 31.7 | 60 | 30.8 |

Table 8 Effect of the kinetic energy water of the present invention on the blood glucose level of the islet-damaged hyperglycemia mice after 30 days of administration (mmol/L , $\bar{x} \pm s$, n=10)

| Group | Blank control | Model group | Low dose kinetic energy water group | High dose kineticenergy water group | Metformin hydrochlor ide | Metformin hydrochloride + kinetic energy water |
|---|---|---|---|---|---|---|
| Blood Glucose | 7.5±0.8 | 28.0±8.7 | 25.2±4.0 | 26.3±8.2 | 19.6±9.5 | 28.2±8.6 |

Remark: * Compared with the model group, p<0.05

It can be seen from Table 7 and Table 8 that the blood glucose level of the blank control group was 7.5 ± 0.8 mmol/L after 30 days of continuous administration. The blood glucose values of 4 of the 10 mice (4/10) in the model group, 0 mice (0/10) in the low dose kinetic energy water group, 1 mice (1/10) in the high dose kinetic energy water group and 0 mouse (0/10) in the metformin hydrochloride group were higher than the upper limit of the glucose meter (33.3 mmol/L) and 4 mice (4/10) in the metformin hydrochloride + kinetic energy water group were higher than the upper limit of the glucose meter (33.3 mmol/L). The blood glucose of animals exceeding the upper limit of measurement was calculated in 33.3 mmol/L, the blood glucose results of each group were 28.0 ± 8.7, 25.2 ± 4.0, 26.3 ± 8.2, 19.6 ± 9.5 and 28.2 ± 8.6 mmol/L respectively. Compared with the model group, the absolute value of the blood glucose of the metformin hydrochloride group decreased significantly, but had no statistically difference.

Table 9 Blood glucose results of each group after 30 days of continuous administration (mmol/L)

| Serial number | Blank control | Serial number | Model group | Serial number | Low dose kinetic energy water group |
|---|---|---|---|---|---|
| 1 | 6.4 | 11 | >33.3 | 21 | 32.1 |
| 2 | 6.5 | 12 | 32 | 22 | 29.1 |
| 3 | 7.9 | 13 | >33.3 | 23 | 19.1 |
| 4 | 6.7 | 14 | 32.4 | 24 | 21.4 |
| 5 | 5.5 | 15 | 31 | 25 | 27.1 |
| 6 | 6.2 | 16 | >33.3 | 26 | 25.4 |
| 7 | 8.2 | 17 | 33 | 27 | 23.4 |
| 8 | 8 | 18 | 30.2 | 28 | 30.9 |
| 9 | 8.4 | 19 | >33.3 | 29 | >33.3 |
| 10 | 9.2 | 20 | 10.9 | 30 | 27.6 |
| Serial number | High dose kinetic energy water group | Serial number | Metformin hydrochloride | Serial number | Metformin hydrochloride + kinetic energy water |
| 31 | 29.7 | 41 | 22.6 | 51 | 28.8 |
| 32 | >33.3 | 42 | 20.2 | 52 | 10.8 |
| 33 | 32.6 | 43 | 13.9 | 53 | 21.9 |
| 34 | 7.7 | 44 | 7.4 | 54 | 20.5 |
| 35 | 32.1 | 45 | 21 | 55 | 20.2 |
| 36 | 31.2 | 46 | 14.2 | 56 | 19.8 |
| 37 | 11.5 | 47 | 9.8 | 57 | 6.1 |
| 38 | 33 | 48 | 25.2 | 58 | 25.7 |
| 39 | 29.4 | 49 | 7.6 | 59 | 16.8 |
| 40 | 18.9 | 50 | 28.3 | 60 | 22.4 |

Table 10 Effect of the kinetic energy water of the present invention on the blood glucose level of the islet-damaged hyperglycemia mice after 40 days of administration (mmol/L , $\bar{x} \pm s$, n=10)

| Group | Blank control | Model group | Low dose kinetic energy water group | High dose kinetic energy water group | Metformin hydrochloride | Metformin hydrochloride + kinetic energy water |
|---|---|---|---|---|---|---|
| Blood Glucose | 7.3±1.2 | 30.3±6.9 | 26.7±4.7 | 25.9±9.6 | 17.0±7.5* | 19.3±6.7* |

Remark: ** Compared with the model group, p<0.01

It can be seen from Table 9 and Table 10 that the blood glucose level of the blank control group was 7.3 ± 1.2 mmol/L after 40 days of continuous administration. The blood glucose values of 4 mice of the 10 (4/10) in the model group, 1 mice (1/10) in the low dose kinetic energy water group, 1 mice (1/10) in the high dose kinetic energy water group were higher than the upper limit of the glucose meter (33.3 mmol/L). Similar situation does not appear in the metformin hydrochloride group and the metformin hydrochloride and kinetic energy water group. The blood glucose of animals exceeding the upper limit of measurement was calculated in 33.3 mmol/L, the blood glucose results of each group were 30.3±6.9, 26.9±4.7, 25.9±9.6, 17.0±7.5**, 19.3±6.7** respectively. Compared with the model group, the absolute value of the blood glucose in the low and high dose groups decreased by 11.2% and 14.5% respectively, but there was no significant difference between the two groups. Compared with the control group, the absolute value of the blood glucose in the metformin hydrochloride group and the metformin hydrochloride and kinetic energy water group significantly decreased and there was statistical difference (** p <0.01).

Based on the above results, under the present experimental conditions, after the islet-damaged hyperglycemia mice induced by alloxan continuously drank the micro-kinetic energy drinking water of the present invention for 40 days compared with the model group, the number of animals whose blood glucose value exceeded the upper limit of 33.3 mmol/L decreased, the absolute value of the blood glucose decreased to a certain extent, this result suggests that the kinetic energy water of the present invention may have reducing effect on the blood glucose of the islet-damaged hyperglycemia mice induced by alloxan. It is also recommended to further adopt animal models that are more consistent with type 2 diabetes to confirm the results.

3. Conclusion

Under the present experimental conditions, after the islet-damaged hyperglycemia mice induced by alloxan continuously drank the micro-kinetic energy drinking water of the present invention for 40 days compared with the model group, the number of animals whose blood glucose value exceeded the upper limit of 33.3 mmol/L decreased, the absolute value of the blood glucose decreased to a certain extent, this result suggests that the kinetic energy water of the present invention may have reducing effect on the blood glucose of the islet-damaged hyperglycemia mice induced by alloxan.

**Example 10 Effect of the multi-polar micro-kinetic energy drinking water of the present invention on the blood glucose of normal animals**

1. Materials and Methods

1.1 Samples:

The multi-polar micro-kinetic energy drinking water (the kinetic energy water) prepared in Example 3, which is a colorless, tasteless and transparent water-like liquid, stored at room temperature and should be used within 72 hours after being prepared.

Jin Aoli auxiliary hypoglycemic capsules, which was obtained from Weihai Nanbowan Biotechnology Co. Ltd., the batch number is 13122201.

1.2 Experimental animals: ICR mice purchased from Beijing Huafu Kang Biotechnology Co. Ltd., the production license number is SCXK (Beijing) 2014-0004, the laboratory animal quality certificate number is 11401300013568. There are 70 in all, all of them are males, the ordering weight is 18-20g.

Feeding conditions: the laboratory animal facilities continue to maintain the barrier environmental standards. The main environmental indicators are as follows: the room temperature is 20 ~ 26 °C, the daily temperature difference is ≤ 4 °C. The relative humidity is 40 to 70%. The minimum ventilation time is 15 times per hour. Lighting: Dark = 12 h: 12 h. Animals were housed in PC mice rearing cages and the specifications of cages were $294 \times 190 \times 125$ mm$^3$, 5 in each cage, and their space was in accordance with the provisions of GB14925-2010 of the People's Republic of China on the minimum required space for experimental animals. All animals are fed and managed by trained and qualified personnel. The padding and cage are replaced once a week. And the special feed for mice is added every day for animal consumption and the animal diet activities are kept free during the whole feeding process.

Animal welfare: the animal and related treatment used in this test should meet the requirements of animal welfare. Experiments were carried out after being reviewed and approved by the Laboratory's Institutional Animal Care and Use Committee (IACUC), Beijing Jianhao Pharmaceutical Technology Development Co. Ltd.. The specific considerations are as follows:

(1) During the course of the experiment, the animal's panic and pain should be minimized.

(2) Effective anesthesia must be performed in the operation and dissection of experimental animals. Analgesic, targeted care and diet conditioning should be performed based on the actual situation during postoperative recovery period.

(3) Animal disturbances, panic, pain and injure should be avoided in the experiments of fixation of animal. Fixation machine should have a reasonable structure, appropriate specification, should be durable, environmentally friendly and easy to operate. Without prejudice to the experiment, the mandatory restrictions on the animal's body should be reduced to a minimum.

(4) Sample collection: the operation of the sample collection of experimental animals should be carried out in a safe and humane manner (to minimize stress and discomfort caused to animals).

(5) Dying animals, diseased animals and animals with severe toxicity should be promptly reported to the veterinarian and the person in charge to timely treat or euthanize. Without affecting the determination of the experiment results, "humane endpoint" should be chosen to avoid prolonging the time when the animals suffer from pain. It should be euthanized in accordance with the humanitarian principles when the animals are sacrificed. At the scene of the execution, there should be no other animals present. The body can be properly disposed of after confirmation of the death of the animal.

(6) Occupational safety: personal protective measures such as gloves, gowns, masks, goggles and earplugs etc. should be taken during the experiment.

1.3 Dose selection and the administering manner of the test substances:

The test was divided into four groups: blank control group, in which the animals daily drank normal drinking water (which was given by gavage once a day with a dose of 0.3 mL/lOg normal drinking water); the low dose kinetic energy water group, in which the animals were given the kinetic energy water daily (the kinetic energy water was kept in a drinking bottle, and supplemented once a day y); the high dose kinetic energy water group, in addition to drinking the kinetic energy water daily, in which the animals were given the kinetic energy water by gavage once a day, the dose was 0.3 ml/10 g; the positive-drug group, in which the animals were given by gavage once a day, the administration dose was 300 mg/kg and the administration volume was 0.3 mL/10 g (the daily dosage of the auxiliary hypoglycemic capsules per person is 4 pills, with a total of 1.6 g, if the weight of a person is 60kg, the dosage on the person is equivalent to 0.03g/kg, if the dosage used in mice is 30 times of that used in the person, the dosage on the mice is equivalent to 0.9 g/kg, the administration volume is 30 mL/lOg and the concentration of drugs is 0.03 g/mL). No fasting water was required during the experiment period and the administration was continued for 29 days.

Table 11 Experimental design of effect of the kinetic energy water of the present invention on the blood glucose levels in normal female mice

| Group | Dosage | Administration volume | Number of animals | Cage number | Serial number |
|---|---|---|---|---|---|
| Blank control | | - | 10 | 1,2 | 1-10 |
| Low dose kinetic energy water group | Daily drinking | Daily drinking | 10 | 3,4 | 11-20 |
| High dose kinetic energy water group | Daily drinking +0.3 mL/10g | Daily drinking +0.3 mL/10g | 10 | 5,6 | 21-30 |
| Auxiliary hypoglycemic capsules | 0.9 g/kg | 0.3mL/10g | 10 | 7,8 | 31-40 |

1.4 Main instruments and reagents:

Roche ACCU-CHEK fast blood glucose meter, special test papers for the blood glucose meter.

1.5 Test methods:

To determine the effects on the blogroupod glucose of normal mice: before administration, the blood glucose of the mice was collected for measurement and the group was divided according to the blood glucose levels to make the blood glucose levels among the groups be no significant difference. After continuous administration for 29 days, the blood samples were taken at 30 min after the last administration and the blood glucose was measured. The blood samples were taken by pricking the tail vein with a needle, and the volume of the collected blood was at least 5 $\mu$L.

1.6 Statistics and analysis of the test data

A SPSS statistical software was used to process the results of body weight and compare and analyze the administration and control group. Statistical analysis was done according to the following methods: the normal test was performed using Kolmogorov-Smirnov method and the test for homogeneity of variance was performed using Levene median method, if P>0.05, then one-way ANOVA method will be performed, and if the normal test and the test for homogeneity of variance failed (P<0.05), then a nonparametric Mann-Whitney test will be required.

1.7 Determination of results

If the blood glucose of the test sample was significantly lower than that of the control group and the difference was significant, the test result can be judged as positive.

2. Results

Table 12 Effects of the kinetic energy water of the present invention on the blood glucose in normal ICR mice (mmol/ L , $\overline{x} \pm s$, n=10)

|  | Blank control | Low dose kinetic energy water group | High-dose kinetic energy water group | Positive drug group |
| --- | --- | --- | --- | --- |
| Blood glucose | 8.3±1.1 | 7.8±0.9 | 8.4±0.7 | 7.2±1.3 |
| before administration Blood glucose after administration for 29 days | 7.4±1.2 | 7.6±1.0 | 7.3±1.0 | 7.2±0.7 |

It can be seen from Table 12 and Figure 10 that after administration for 29 days, there was no significant difference among the low dose kinetic energy water group, the high-dose kinetic energy water group and the blank control group, and their absolute values were consistent; there was no significant difference between the positive-drug Jin Aoli auxiliary hypoglycemic capsules and the blank control group. It is suggested that the kinetic energy water of the presen invention and Jin Aoli auxiliary hypoglycemic capsules have no significant effect on the blood glucose in normal mice.

3. Conclusions

After continuously drinking the kinetic energy water for 29 days, the blood glucose results in normal ICR mice do not have statistically different from that of blank control group, their absolute values were consistent.
The kinetic energy water has no significant effect on the blood glucose in normal mice.

**Claims**

**1.**  A preparation method of a drinking water, comprising:

applying three different frequencies of electromagnetic waves: low, medium and high frequency electromagnetic waves, at the outside of the pipe of raw water of ordinary drinking water in non-contact mode, wherein the frequency of the low frequency electromagnetic wave is 45 kHz, the frequency of the medium frequency electromagnetic wave is 600 kHz, and the frequency of the high frequency electromagnetic wave is 300 MHz; wherein, the low frequency electromagnetic wave and the medium frequency electromagnetic wave are both sine wave and they are propagated in the same direction, the high frequency electromagnetic wave is triangular wave, and its propagation direction is perpendicular to that of the low frequency electromagnetic wave and the medium frequency electromagnetic wave, wherein during the treatment, firstly, the low frequency electromagnetic wave and the high frequency electromagnetic wave are used simultaneously for 15 minutes, and then the medium electromagnetic wave and the high frequency electromagnetic wave are used simultaneously for 15 minutes.

**Patentansprüche**

**1.**  Ein Herstellungsverfahren für Trinkwasser umfassend die Anwendung von drei verschiedenen Frequenzen von elektromagnetischen Wellen an der Außenseite des Rohwasserrohres von gewöhnlichem Trinkwasser im berührungslosem Modus, wobei die Frequenz der niederfrequenten elektromagnetischen Welle 45 kHz ist, die Frequenz der mittelfrequenten elektromagnetischen Welle 600 kHz ist und die Frequenz der hochfrequenten elektromagnetischen Welle 300 MHz ist; wobei die niederfrequente Welle und die mittelfrequente elektromagnetische Welle beides Sinuswellen sind und in dieselbe Richtung verbreitet werden, die hochfrequente Welle ist eine Dreieckswelle und ihre Verbreitungsrichtung ist perpendikular zu der der niederfrequenten Welle und zu der der mittelfrequenten Welle, wobei während der Behandlung, erstens die niederfrequente und die hochfrequente elektromagnetische Welle simultan für 15 Minuten und dann die mittelfrequente Welle und die hochfrequente Welle simultan für 15 Minuten angewandt werden.

**Revendications**

1. Procédé de préparation d'une eau potable, comprenant:

l'application de trois fréquences différentes d'ondes électromagnétiques : ondes électromagnétiques de basse, moyenne et haute fréquence, à l'extérieur du tuyau d'eau brute d'eau potable ordinaire en mode sans contact, dans lequel la fréquence de l'onde électromagnétique de basse fréquence est de 45 kHz, la fréquence de l'onde électro-magnétique de moyenne fréquence est de 600 kHz, et la fréquence de l'onde électro-magnétique de haute fréquence est de 300 MHz;
dans lequel, l'onde électromagnétique à basse fréquence et l'onde électro-magnétique à moyenne fréquence sont toutes deux des ondes sinusoïdales et se propagent dans la même direction, l'onde électromagnétique à haute fréquence est une onde triangulaire, et sa direction de propagation est perpendiculaire à celle de l'onde électromagnétique à basse fréquence et de l'onde électromagnétique à moyenne fréquence, dans lequel, pendant le traitement, l'onde électromagnétique à basse fréquence et l'onde électromagnétique à haute fréquence sont d'abord utilisées simul-tanément pendant 15 minutes, puis l'onde électromagnétique à moyenne fréquence et l'onde électromagnétique à haute fréquence sont utilisées simultanément pendant 15 minutes.

Group I    Group II    Group III

1kb    Plasmid  0.5h 1h    0.5h 1h    0.5h 1h
marker DNA

Figure 1

Treated group: culture medium A    Untreated group: culture medium B

Figure 2

Figure 3

Figure 4

Figure 5

Figure 6

Figure 7

Figure 8

Figure 9

Groups

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090242407 A1
- US 2007221577 A1
- US 20040050682 A1